# EUROPEAN PATENT APPLICATION

(11) **EP 2 679 145 A1**
(43) Date of publication of application: **01.01.2014**
(21) Application number: 12715119.9
(22) Date of filing: 23.02.2012
(51) Int. Cl.: A61B 1/267, A61B 1/00

(54) **LARYNGOSCOPY DEVICE**

(30) Priority: 24.02.2011 ES 201130246
(71) Applicant: Domenech Oliva, Juan, 08037 Barcelona (ES)
(72) Inventor: Domenech Oliva, Juan, 08037 Barcelona (ES)
(74) Representative: Curell Suñol S.L.P.
(86) International application number: PCT/ES2012/070107
(87) International publication number: WO 2012/113961

(57) **Abstract**

An accessory for laryngoscopy. An accessory for laryngoscopy that comprises
[a] a bar (7), having a first end and a second end,
[b] support means (13) at the first end of the bar (7) adapted to bear against a larynx of a patient (who has to be subjected to an examination or surgical operation on this zone),
[c] retaining means (15) adapted to attach the accessory to a second bar (39),
[d] length adjusting means (17) adapted to adjust the distance between the support means (13) and the retaining means (15).

The accessory allows a downward pressure to be exerted in a continuous controlled way from outside of the neck on the laryngeal zone. In said way it becomes possible to move the larynx, which remarkably improves the exposure of the anterior commissure of the larynx and the adjacent area.

## Description

### Field of the Invention

The invention relates to an accessory for laryngoscopy.

### State of the Art

The larynx is the organ of the voice and is situated at the upper end of the air passage (the trachea) that leads to the lungs. It is disposed just under the base of the tongue. It is composed of cartilages, muscles and ligaments, which allow for the movements that produce the human voice and permit breathing. The main cartilage is the thyroid, which stands in the middle of the neck and forms the laryngeal prominence which can be felt from the outside. It has roughly the form of an open book with the pages facing backwards and it contains the vocal cords.

The vocal cords are two muscular and mucous folds which are placed in the airway inside the thyroid cartilage and move in an open-close movement, allowing for breathing (in the open position thereof) or speech (in the closed position). The space between the cords and, in general, the entire zone including the cords, is the glottis. The inside of the larynx is covered with a mucosa which may suffer from various diseases, such as nodules, polyps, inflammations of various types, and tumours, both benign and malignant.

Direct laryngoscopy, also called suspension laryngoscopy is a technique allowing for direct examination of the inside of the larynx as well as the performance of various surgical procedures. It is the technique of choice for taking biopsies and for removing laryngeal tumours and is the most common procedure performed on the larynx under general anesthesia. A laryngoscope is used to this end, being a hollow and slightly conical tube, which is introduced in the mouth of the anesthetized patient and is inserted until the inside of the larynx is correctly visualized. Next, by means of a surgical microscope and suitable surgical instruments biopsies can be taken or pathological lesions can be removed. The laryngoscope is held in the correct position with the aid of a lever which, on the one hand, is attached to the laryngoscope and, on the other side, rests over the chest of the patient (or, possibly, on any accessory surface present in the operating theatre). In this way an upward pressure can be obtained at the end of the laryngoscope, allowing the zone known as the anterior commissure, which is the zone where the two vocal cords meet in the anterior part of the glottis, to be seen.

One of the problems presented by this procedure is that the visualization of the anterior commissure is, in certain cases, very difficult due to anatomical reasons. For this reason, in certain cases it is necessary for a downward force to be exerted from the outside of the neck, allowing the anterior commissure to fall within the field of vision facilitated by the laryngoscope. Usually an assistant is used to manually press the larynx until a good view is obtained.

### Summary of the Invention

It is an object of the invention to overcome these drawbacks. This purpose is achieved by means of a laryngoscopy accessory characterized in that it comprises
[a] a bar, having a first end and a second end,
[b] support means at the first end of the bar adapted to bear against a larynx of a patient (who has to be subjected to an examination or surgical operation on this zone),
[c] retaining means adapted to attach the accessory to a second bar,
[d] length adjusting means adapted to adjust the distance between the support means and the retaining means.

In fact, the accessory according to the invention is therefore a mechanical apparatus allowing a downward pressure to be exerted in a continuous and controlled way from outside of the neck on the laryngeal zone. In this way it is possible to move the larynx, which remarkably enhances the exposure of the laryngeal anterior commissure and the adjacent area. This is particularly important since this part of the larynx is prone to develop malignant tumours, which may be underestimated by the surgeon, due precisely to an incomplete exposure of the area at issue. The accessory allows the assistant's hand to be replaced, thus releasing this person for other tasks and also avoiding the small movements which are unavoidable if the pressure is exerted manually. This is particularly important if a surgical laser is used, since this device must be used with extreme precision on the laryngeal structures.

The retaining means are attached to an external bar (called the "second bar"), namely a bar foreign to the accessory itself. This second bar may be any one since simply its function is to serve as a fixed support for the accessory. A preferred solution consists of using the lever of the laryngoscope as second bar, as will be shown hereinafter. Another preferred solution consists of using any other bar of any other accessory used during the surgical operation, which is appropriate for fixing the accessory. Once the accessory has been attached to the second bar, it is only necessary to adjust the length so that the support means bear against the patient's neck, on the zone corresponding to the larynx (commonly known as the "Adam's apple") with an appropriate pressure.

Preferably the support means are attached to the bar by means of a ball and socket joint. The ball and socket joint allows the relative position between the support means and the bar to be modified. Indeed, in certain cases it is desirable for the pressure exerted on the larynx not to be vertical but that its orientation can be modified so that it is more or less inclined. In this way it is possible to better adjust the zone visible through the laryngoscope depending on the particular point to which access is required. Advantageously the inclination of the support means can be varied relative to the axis defined by the bar in at least 30º in any direction. Also, in this way any positioning of the bar on the second bar that is not exactly the required one can be corrected. Preferably the ball and socket joint is closer to the support means than to the second end. In fact, in this way the ball and socket joint can be more precisely positioned relative to the larynx (since it is closer thereto).

A preferred embodiment of the retaining means is that they comprise clamps adapted to be disposed around the second bar and tightening means adapted to tighten the clamps on the second bar. Alternatively, the retaining means may comprise a part with at least one groove adapted to receive, at least partially, the second bar. Since the accessory will work under compressive stress, one groove may be sufficient for the accessory to be fixed between the second bar and the patient's larynx.

Advantageously the accessory comprises rotary means that allow the bar to be rotated relative to the retaining means according to an axis of rotation inclined relative to the second bar, preferably perpendicular to the second bar. Indeed, in this way no precise positioning of the accessory relative to the second bar is necessary but it may be placed in an approximate position and, although it is not located perpendicular between the second bar and the larynx, it may be oriented so that the support means are in contact with the larynx. Preferably these rotary means are associated with the afore-mentioned ball and socket joint, since the combination of both allows great freedom in the positioning and orientation of the accessory.

The length adjusting means may adopt different forms. A preferred form consists of the length adjusting means comprising clamps disposed around the bar and tightening means to tighten the clamps on the bar. This simple geometry allows the bar to be moved between the clamps until the correct position is reached in order, thereafter, to tighten the clamps by means of the tightening means, which may, for example, be a threaded screw.

A particularly advantageous embodiment of the accessory is obtained when the retaining means and the length adjusting means are formed by two first plates and two second plates, all adjacent to each other, where the two first plates comprise first grooves adapted to receive the bar and the two second plates comprise second grooves adapted to receive the second bar, where the two first plates and the two second plates are all crossed by one same tightening screw, and where the two first plates are adapted to rotate relative to the two second plates according to an axis of rotation defined by the tightening screw when the tightening screw is loosened. This particular embodiment will be shown in greater detail in the figures.

Preferably the accessory comprises second length adjusting means, where the second length adjusting means are adapted for a more precise adjustment of the length than the length adjusting means. Indeed, this is particularly interesting to be able to control more accurately the pressure exerted on the larynx. During the installation of the accessory it is likely that the length must be adjusted over a relatively long distance, for example several centimetres, whereas the pressure control exerted on the larynx may require a quite precise adjustment of the distance, of the order of millimetres. Possibly length adjusting means suitable for adjusting several centimetres in an agile way are not very appropriate for making fine adjustments of a few millimetres. On the contrary, length adjusting means adapted for adjusting the length with a precision of a few millimetres may be very slow if distances of several centimetres have to be adjusted. In this sense, the fact of being able to have two length adjusting means, one allowing for the approximate positioning of the support means to the larynx, for example until it touches the patient's neck, and others allowing the pressure exerted on the larynx to be accurately adjusted, means an advantage and a greater versatility for the accessory.

Advantageously, the bar has a rack and one of the adjusting means or the second length adjusting means has a pinion engaging the rack and adapted to rotate about itself, while moving along the rack. This system can be appropriate both for the adjusting means and for the second adjusting means, since its accuracy will depend on the size of the rack, of the geared pinion and the handle (for example a wheel or the like) associated with the geared pinion and by means of which the geared pinion is rotated. In fact, the accessory could comprise two racks and two pinions, designed to perform, on the one hand, a fast adjustment and, on the other hand, a fine adjustment.

Preferably the bar is divided into two coaxial sections and the second length adjusting means are adapted to make a relative displacement between both sections. Indeed, in this way the section of the bar which is attached to the length adjusting means becomes mechanically independent from the section of the bar which is attached to the support means. Advantageously one of the coaxial sections is a tube and the other coaxial section is a threaded stud, where the tube is adapted to house, at least partially, the stud, where the second length adjusting means comprise a nut coaxial to the tube, non-removably attached to the tube but adapted to rotate relative to the tube, and where the stud is threaded into the nut. In this way the stud may be moved relative to the tube by rotating the nut. The threaded stud may have the finest thread required to allow a suitable adjustment of the pressure exerted on the larynx.

Both the afore-mentioned length adjusting means and the second length adjusting means are preferred examples of embodiments, but are not the only ones since other mechanical length adjustment systems, such as, for example, lever sets, etc. could be applied.
Preferably the support surface is a surface having an anatomical geometry adapted to adjust itself to a human neck in the zone corresponding to the larynx. In this sense, the support surface is a cylindrical surface. In the present description and claims a cylindrical surface should be understood to be any surface with a straight line generatrix generated when moving a straight line parallel to itself along a curved or broken line. In this sense, the cylindrical surfaces having a circular, elliptical section, etc. but also the prismatic surfaces, obtained when moving a straight line, parallel to itself, along a broken line must be included within the cylindrical surfaces. Advantageously the support surface is formed by two planes forming together an angle of between 60º and 120º, and very preferably is formed by two planes forming together an angle of between 80º and 100º. Indeed, it has been observed that these angles are particularly adapted to adjust to the external surface of the larynx, 90º being the angle that is best adapted for the majority of cases.

Preferably the support surface is covered by a detachable or interchangeable sheet. Indeed, it is this surface that finally exerts the pressure on the patient's neck in the zone corresponding to the larynx. Since this support surface is the one that is in direct contact with the patient, providing it with an interchangeable sheet facilitates its cleaning and asepsis. In addition, preferably this sheet is made from a soft material, appropriate for making contact with the patient's skin, such as for example silicone. It must be borne in mind that the support surface will exert a certain pressure whereby is desirable to avoid possible injuries to the skin of the patient's neck.

### Brief Description of the Drawings

Further advantages and characteristics of the invention will be appraised from the following description, in which, without any limiting character, preferred embodiments of the invention are disclosed, with reference to the accompanying drawings, in which:
Fig. 1 is a schematic view of a conventional laryngoscope placed in the position of use.
Fig. 2 is a perspective view of a first embodiment of an accessory according to the invention.
Fig. 3 is a perspective view of a second embodiment of an accessory according to the invention.
Fig. 4 is a perspective view of the accessory of Fig. 2 mounted on the laryngoscope lever.
Fig. 5 is a perspective view of a third embodiment of an accessory according to the invention.
Fig. 6 is a perspective view of the accessory of Fig. 5 mounted on an auxiliary bar.
Fig. 7 is a partial front view and partially in section of the accessory of Fig. 5.

### Detailed Description of Embodiments of the Invention

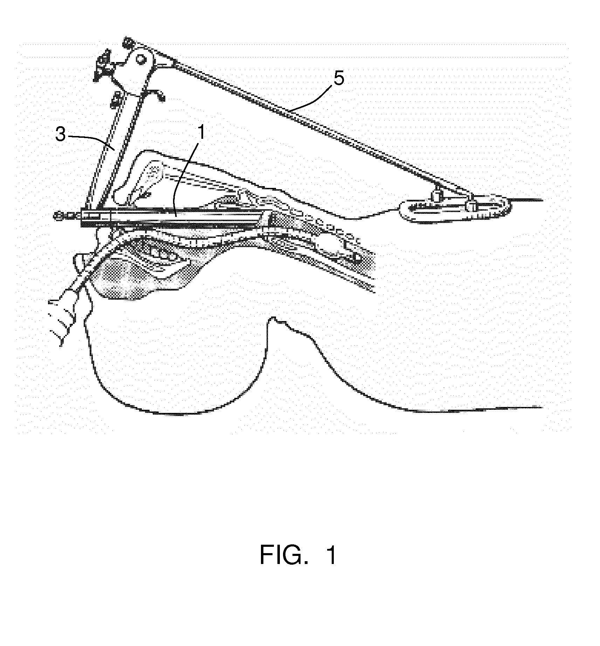

In Fig. 1 there is shown a conventional laryngoscope in the position of use. It has a hollow, slightly conical tube 1 that is inserted in the patient's mouth and extends to the larynx. It has a handle 3 allowing it to be manipulated and a lever 5, attached to the handle 3, the opposite end of which bears on the patient's chest.

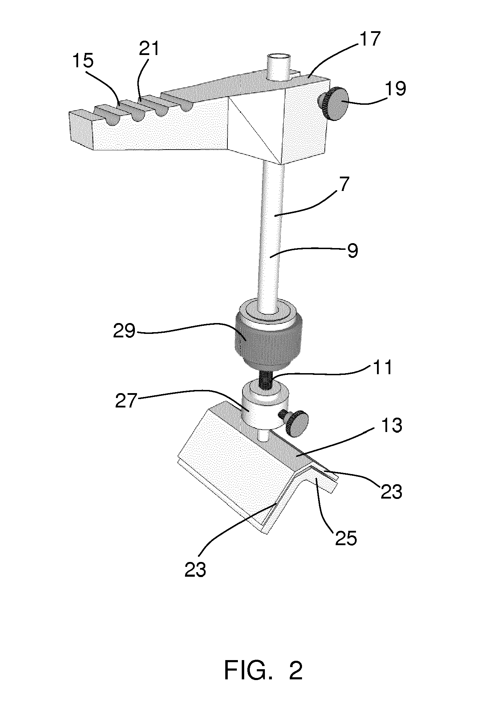

In Fig. 2 there is shown a first embodiment of an accessory according to the invention. It comprises a bar 7 that is divided into two coaxial sections; the first section is a tube 9 and the second section is a stud 11. The bar 7 has a first end having support means 13 and a second end having retaining means 15 and length adjusting means 17. In this particular embodiment the retaining means 15 and the length adjusting means 17 are constituted by one same member, that comprises a through hole in which the second end of the bar 7 is received so that the bar 7 can slide through the through hole to the desired position. A screw 19 allows the position of the bar 7 to be set thereby allowing a first adjustment of the distance between the support means 13 and the retaining means 15. The member also includes a plurality of grooves 21 that will serve as retaining means 15. The support means 13 comprise a prismatic member with two main faces or planes 23 that define the support surface and which form therebetween an angle of 90º. The support means 13 also include a quilted interchangeable sheet 25. Adjacent the support means 13 there is a ball and socket joint 27 allowing for free orientation of the support means 13 relative to the bar 7. The ball and socket joint 27 is attached to the stud 11. Between the stud 11 and the tube 9 there are second length adjusting means 29.

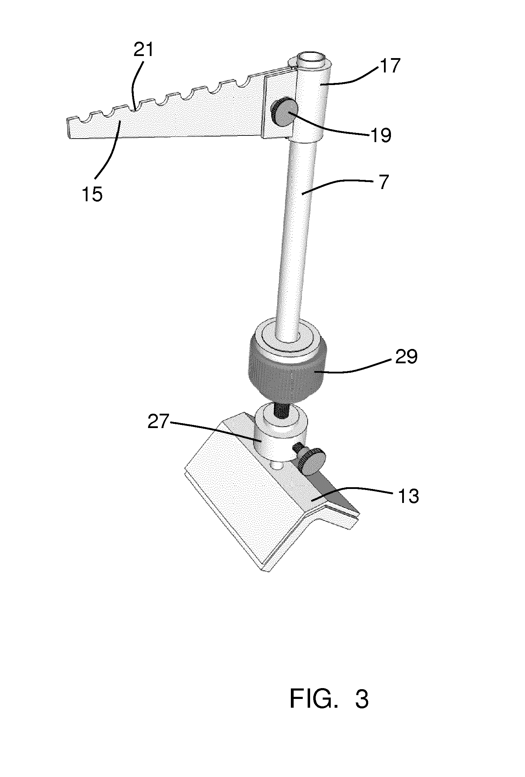

In Fig. 3 there is shown a second embodiment of an accessory according to the invention. The difference relative to the first embodiment is to be found in the member functioning as the retaining means 15 and length adjusting means 17. Indeed, in this case it is not a member having a certain thickness but a thin sheet having the grooves 21 and bent through 180º, forming the through hole in which the bar 7 is received.

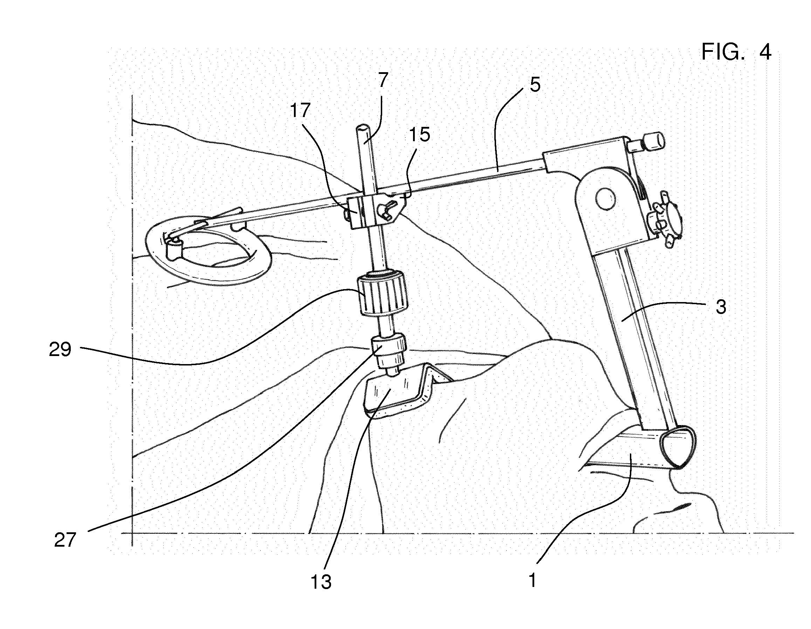

In Fig. 4 there is shown a laryngoscope placed in the position of use and an accessory such as the one shown in Fig. 2. The accessory receives the lever 5 of the laryngoscope in one of its grooves 21 (having the function of the second bar 39). The support means 13 bear against the patient's larynx while exerting a certain pressure. This pressure also serves for the accessory to be retained on the laryngoscope lever 5.

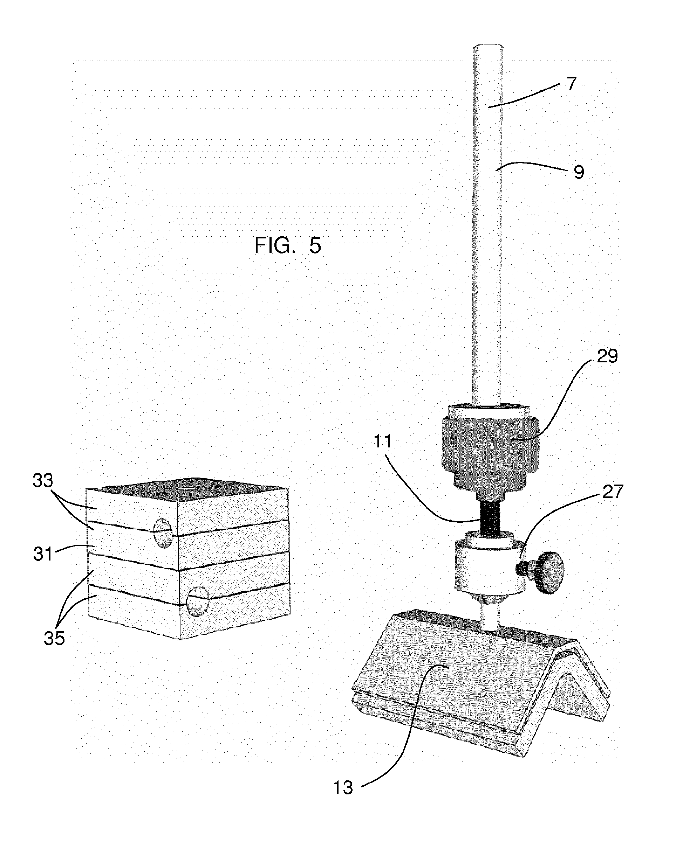

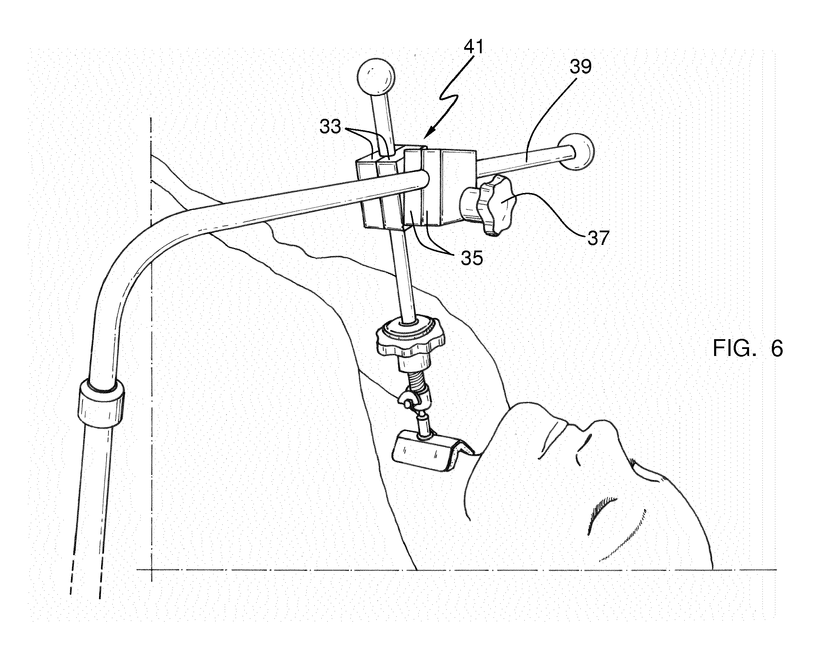

In Fig. 5 there is shown a third embodiment of an accessory according to the invention. It comprises various members in common with the embodiments of Figs. 2 and 3, such as are the bar 7 that comprises a tube 9 and a stud 11 with second length adjusting means 29 between both, the ball and socket joint 27 and the support means 13. The difference is to be found in the retaining means 15 and the length adjusting means 17. In this case these two functions are performed by four plates 31 shown at the left of the figure, disassembled relative to the bar 7. The plates 31 have two first plates 33 having first grooves appropriate for receiving the bar 7, and two second plates 35 having second grooves appropriate for receiving a second bar, that can be the laryngoscope lever 5 or any other auxiliary bar present on the surgical table. The four plates 31 are connected to each other by means of a tightening screw 37, not shown in Fig. 5. Fig. 6 shows this third embodiment of the accessory mounted on an auxiliary bar (acting as the second bar 39). It may be seen how the four plates 31 fulfill the function of length adjusting means 17, of retaining means 15 and, additionally, the two first plates 33 can rotate relative to the two second plates 35, so that they additionally form rotary means 41.

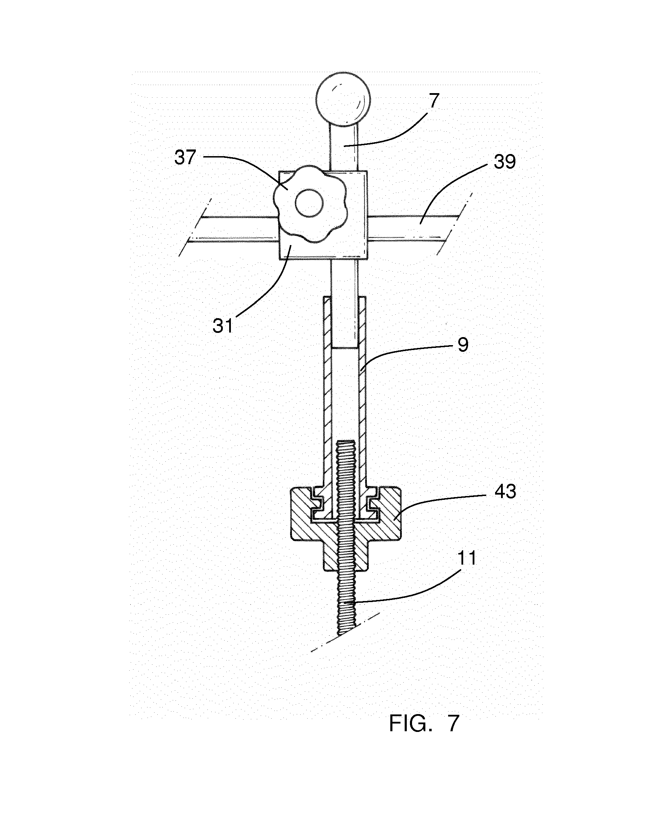

In Fig. 7 there is shown an exemplary embodiment of the second length adjusting means 29. As already stated above the bar 7 comprises a tube 9 and a stud 11. The second length adjusting means 29 have a nut 43 coaxial with the tube 9 and non-removably attached to the tube 9 but adapted to rotate relative to the tube 9. In this way, when the nut 43 is rotated a relative movement between the tube 9 and the stud 11 can be obtained.

## Claims

1. An accessory for laryngoscopy **characterized in that** it comprises
[a] a bar (7), having a first end and a second end,
[b] support means (13) at said first end of said bar (7) adapted to bear against a patient's larynx,
[c] retaining means (15) adapted to attach said accessory to a second bar (39),
[d] length adjusting means (17) adapted to adjust the distance between said support means (13) and said retaining means (15).

2. The accessory according to claim 1, **characterized in that** said support means (13) are attached to said bar (7) by means of a ball and socket joint (27).

3. The accessory according to claim 2, **characterized in that** said ball and socket joint (27) is closer to said support means (13) than to said second end.

4. The accessory according to anyone of claims 1 to 3, **characterized in that** said retaining means (15) comprise clamps adapted to be disposed around said second bar (39) and tightening means adapted to tighten said clamps on said second bar (39).

5. The accessory according to anyone of claims 1 to 3, **characterized in that** said retaining means (15) comprise a member with at least one groove (21) adapted to receive, at least partially, said second bar (39).

6. The accessory according to anyone of claims 1 to 5, **characterized in that** it comprises rotary means (41) allowing said bar (7) to be rotated relative to said retaining means (15) according to an axis of rotation inclined relative to said second bar (39), preferably perpendicular to said second bar (39).

7. The accessory according to anyone of claims 1 to 6, **characterized in that** said length adjusting means (17) comprise clamps disposed around said bar (7) and tightening means to tighten said clamps on said bar (7).

8. The accessory according to claim 1, **characterized in that** said retaining means (15) and said length adjusting means (17) are formed by two first plates (33) and two second plates (35), all adjacent to each other, where said two first plates (33) comprise first grooves adapted to receive said bar (7) and said two second plates (35) comprise second grooves adapted to receive said second bar (39), where said two first plates (33) and said two second plates (35) are all crossed by one same tightening screw (37), and where said two first plates (33) are adapted to rotate relative to said two second plates (35) according to an axis of rotation defined by said tightening screw (37) when said tightening screw (37) is loosened.

9. The accessory according to anyone of claims 1 to 8, **characterized in that** it comprises second length adjusting means (29), where said second length adjusting means (29) are adapted for a more precise adjustment of said length than said length adjusting means (17).

10. The accessory according to anyone of claims 1 to 9, **characterized in that** said bar (7) has a rack and one of said adjusting means or said second length adjusting means (29) has a pinion engaged with said rack and adapted to rotate about itself, moving on said rack.

11. The accessory according to one of claims 9 or 10, **characterized in that** said bar (7) is divided into two coaxial sections and said second length adjusting means (29) are adapted for relative movement between both sections.

12. The accessory according to claim 11, **characterized in that** one of said coaxial sections is a tube (9) and the other of said coaxial sections is a threaded stud (11), where said tube (9) is adapted to receive, at least partially, said stud (11), where said second length adjusting means (29) comprise a nut (43) coaxial to said tube (9), non-removably attached to said tube (9) but adapted to rotate relative to said tube (9), and where said stud (11) is threaded to said nut (43).

13. The accessory according to anyone of claims 1 to 12, **characterized in that** said support surface is a surface having anatomical geometry adapted to adjust itself to a human neck on the zone corresponding to the larynx.

14. The accessory according to claim 13, **characterized in that** said support surface is a cylindrical surface, preferably is formed by two planes (23) forming therebetween an angle of between 60º and 120º, and most preferably is formed by two planes (23) that form therebetween an angle of between 80º and 100º.

15. The accessory according to anyone of claims 1 to 14, **characterized in that** said support surface is covered by a removable or interchangeable sheet (25).
